(19) Europäisches Patentamt
European Patent Office
Office européen des brevets

(11) Veröffentlichungsnummer: **0 031 021**
**B1**

# (12) EUROPÄISCHE PATENTSCHRIFT

(45) Veröffentlichungstag der Patentschrift: **18.05.83**

(51) Int. Cl.³: **C 07 D 239/74,** C 09 K 3/34

(21) Anmeldenummer: **80106928.7**

(22) Anmeldetag: **10.11.80**

(54) **Tetrahydrochinazoline, Verfahren zu ihrer Herstellung, diese enthaltende flüssigkristalline Dielektrika und elektrooptisches Anzeigeelement.**

(30) Priorität: **19.12.79 DE 2951099**

(43) Veröffentlichungstag der Anmeldung: **01.07.81 Patentblatt 81/26**

(45) Bekanntmachung des Hinweises auf die Patenterteilung: **18.05.83 Patentblatt 83/20**

(84) Benannte Vertragsstaaten: **AT CH DE FR GB IT LI NL**

(56) Entgegenhaltungen:
**DE-A-2 257 588**
**DE-A-2 547 737**

(73) Patentinhaber: **Merck Patent Gesellschaft mit beschränkter Haftung, Frankfurter Strasse 250, D-6100 Darmstadt (DE)**

(72) Erfinder: **Krause, Joachim, Dr., Samuel-Morse-Strasse 14, D-6110 Dieburg (DE)**
Erfinder: **Pohl, Ludwig, Dr., Niebergallweg 5, D-6100 Darmstadt (DE)**

**EP 0 031 021 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Tetrahydrochinazoline, Verfahren zu ihrer Herstellung, diese enthaltende flüssigkristalline Dielektrika und elektrooptisches Anzeigeelement

Für elektrooptische Anzeigeelemente werden in zunehmendem Masse die Eigenschaften nematischer oder nematisch-cholesterischer flüssigkristalliner Materialien ausgenutzt, ihre optischen Eigenschaften wie Lichtabsorption, Lichtstreuung, Doppelbrechung, Reflexionsvermögen oder Farbe unter dem Einfluss elektrischer Felder signifikant zu verändern. Die Funktion derartiger Anzeigeelemente beruht dabei beispielsweise auf den Phänomenen der dynamischen Streuung, der Deformation aufgerichteter Phasen, dem Schadt-Helfrich-Effekt in der verdrillten Zelle oder dem cholesterisch-nematischen Phasenübergang.

Für die technische Anwendung dieser Effekte in elektronischen Bauelementen werden flüssigkristalline Dielektrika benötigt, die einer Vielzahl von Anforderungen genügen müssen. Besonders wichtig sind hier die chemische Beständigkeit gegenüber Feuchtigkeit, Luft und physikalischen Einflüssen wie Wärme, Strahlung im infraroten, sichtbaren und ultravioletten Bereich und elektrische Gleich- und Wechselfelder. Ferner wird von technisch verwendbaren flüssigkristallinen Dielektrika eine flüssigkristalline Mesophase im Temperaturbereich von mindestens +10 °C bis +50 °C, bevorzugt von 0° bis 60 °C, und eine möglichst niedrige Viskosität bei Raumtemperatur, die vorzugsweise nicht mehr als $70 \cdot 10^{-3}$ Pa.s betragen soll, gefordert. Schliesslich dürfen sie im Bereich des sichtbaren Lichtes keine Eigenabsorption aufweisen, d.h. sie müssen farblos sein.

Es ist bereits eine Anzahl von flüssigkristallinen Verbindungen bekannt, die den an Dielektrika für elektronische Bauelemente gestellten Stabilitätsanforderungen genügen und auch farblos sind. Hierzu gehören insbesondere die in der DE-OS 2 139 628 beschriebenen p,p'-disubstituierten Benzoesäurephenylester und die in der DE-OS 2 636 684 beschriebenen p,p'-disubstituierten Phenylcyclohexanderivate. In beiden genannten Verbindungsklassen wie auch in anderen bekannten Reihen von Verbindungen mit flüssigkristalliner Mesophase gibt es keine Einzelverbindungen, die in dem geforderten Temperaturbereich von 10 °C bis 60 °C eine flüssigkristalline nematische Mesophase ausbilden. Es werden daher in der Regel Mischungen von zwei oder mehreren Verbindungen hergestellt, um als flüssigkristalline Dielektrika verwendbare Substanzen zu erhalten. Hierzu mischt man gewöhnlich eine Verbindung mit niedrigem Schmelz- und Klärpunkt mit einer anderen mit deutlich höherem Schmelz- und Klärpunkt. Hierbei wird normalerweise ein Gemisch erhalten, dessen Schmelzpunkt unter dem der niedriger schmelzenden Komponente liegt, während der Klärpunkt zwischen den Klärpunkten der Komponenten liegt. Optimale Dielektrika lassen sich jedoch auf diese Weise nicht leicht herstellen, da die Komponenten mit den hohen Schmelz- und Klärpunkten den Gemischen häufig auch eine hohe Viskosität verliehen. Dadurch werden die Schaltzeiten der damit hergestellten elektro-optischen Anzeigeelemente in unerwünschter Weise verlängert.

Ferner sind aus den DE-OS 22 57 588 und 25 47 737 bereits flüssigkristalline Verbindungen aus der Reihe der Phenylpyrimidine, also mit jeweils 2 Stickstoffatome enthaltenden heteroaromatischen Ringen bekannt. Es hat sich jedoch gezeigt, dass diese Verbindungen nur sehr hochviskose flüssigkristalline Mesophasen besitzen und auch in Mischungen mit anderen Flüssigkristallen eine für die breite Verwendung als Dielektrika in elektrooptischen Anzeigeelementen zu hohe Viskosität induzieren. Dies gilt in besonderem Masse dann, wenn in solchen Dielektrika noch zusätzlich klärpunktserhöhende Komponenten verwendet werden.

Der Erfindung liegt die Aufgabe zugrunde, flüssigkristalline Dielektrika herzustellen, die eine nematische Phase im geforderten Temperaturbereich aufweisen und in Flüssigkristallzellen bei Raumtemperatur ausreichend kurze Schaltzeiten ermöglichen.

Es wurde nun gefunden, dass die Tetrahydrochinazoline der Formel (I)

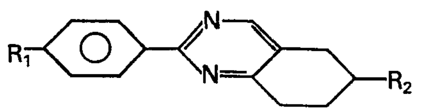

(I)

worin $R_1$ Alkyl, Alkoxy, Alkanoyloxy oder Alkoxycarbonyloxy mit jeweils bis zu 8, vorzugsweise bis zu 6, Kohlenstoffatomen, CN, $CF_3$, Halogen oder $NO_2$ und $R_2$ Alkyl mit bis zu 10 Kohlenstoffatomen bedeutet, vorzüglich als Komponenten flüssigkristalliner Dielektrika geeignet sind. Dabei werden sie insbesondere zur Erweiterung des Temperaturbereichs der nematischen Phase verwendet, weil sie hohe Klärpunkte in Verbindung mit für diesen Strukturtyp überraschend niedrigen Schmelzpunkten besitzen. Ferner weisen die Verbindungen der Formel (I) eine positive dielektrische Anisotropie (DKA) auf, die in Abhängigkeit von der Natur der Flügelgruppen $R_1$ und $R_2$ mehr oder weniger stark ausgeprägt ist. Die Verbindungen der Formel (I) dienen daher vor allem zur Modifizierrung der DKA flüssigkristalliner Dielektrika; besonders die Verbindungen der Formel (I), in denen $R_1$ eine polare Gruppe wie CN, $CF_3$, Halogen oder $NO_2$ ist, besitzen eine erstaunlich hohe positive DKA und werden daher bevorzugt in solchen Dielektrika eingesetzt, die für elektrooptische Anzeigeelemente nach dem Prinzip der verdrillten nematischen Zelle (TNC) oder des cholesterisch-nematischen Phasenübergangs verwendet werden; in derartigen Anzeigeelementen ermöglichen sie die Visualisierung von Informationen mit sehr niedrigen Schwellen- bzw. Betriebsspannungen.

Die Verbindungen der Formel (I) sind in reinem Zustand farblos, chemisch und photochemisch

sehr beständig und bilden allein oder in Kombination mit anderen flüssigkristallinen Substanzen nematische Mesophasen mit erstaunlich niedriger Viskosität.

Gegenstand der Erfindung sind somit die Tetrahydrochinazoline der Formel (I), ihre Herstellung sowie ihre Verwendung als Komponenten flüssigkristalliner Dielektrika. Gegenstand der Erfindung sind weiterhin flüssigkristalline Dielektrika mit einem Gehalt an mindestens einem Tetrahydrochinazolin der Formel (I) sowie elektrooptische Anzeigeelemente auf der Basis einer Flüssigkristallzelle, die ein derartiges flüssigkristallines Dielektrikum enthalten.

In den erfindungsgemässen Verbindungen können die als Flügelgruppen $R_2$ und gegebenenfalls $R_1$ fungierenden Alkylgruppen beziehungsweise die Alkylteile der als Flügelgruppe $R_1$ fungierenden Alkoxy-, Alkanoyloxy oder Alkoxycarbonyloxygruppen geradkettig oder verzweigt sein. In jedem Fall enthalten die erfindungsgemässen Verbindungen jedoch nur höchstens eine verzweigte Alkylgruppe; bevorzugt sind jedoch unter den erfindungsgemässen Verbindungen mit 2 kettenförmigen Flügelgruppen diejenigen, in denen diese beiden Flügelgruppen geradkettig sind, weil die Verbindungen dann in der Regel deutlich höhere Klärpunkte besitzen. Erfindungsgemässe Verbindungen der Formel (I) mit einer verzweigten Flügelgruppe $R_1$ oder $R_2$ sind gelegentlich wegen einer höheren Löslichkeit in den üblichen flüssigkristallinen Basismaterialien von Bedeutung, insbesondere aber als chirale Dotierstoffe, wenn sie durch die Kettenverzweigung optische Aktivität besitzen. Solche verzweigten Substituenten enthalten nicht mehr als eine Kettenverzweigung. Bevorzugte verzweigte Reste sind die, in denen sich an einer längeren Kohlenstoffkette in 2- oder 3-Stellung eine Methyl- oder Ethylgruppe befindet, beispielsweise 2-Methylpropyl, 2-Methylbutyl, 3-Methylbutyl, 2-Methylpentyl, 3-Methylpentyl, 2-Ethylhexyl oder 2-Methylhexyl. Die Flügelgruppen der erfindungsgemässen Verbindungen können definitionsgemäss zusammen bis zu 18 Kohlenstoffatome enthalten. Unter diesen sind die Verbindungen bevorzugt, worin die Flügelgruppen zusammen 2 bis 14, insbesondere 3 bis 12 Kohlenstoffatome enthalten.

Wenn die Flügelgruppe $R_1$ Halogen, $CF_3$, $NO_2$ oder vorzugsweise CN bedeutet, besitzen die so charakterisierten erfindungsgemässen Verbindungen eine besonders stark ausgeprägte positive DKA, die Werte bis zu +35 erreicht. Verbindungen aus dieser Gruppe werden bevorzugt zur Erhöhung der DKA verwendet, beispielsweise anstelle des gemäss der DE-PS 2 321 632 zu diesem Zweck eingesetzten 4-Dimethylaminobenzonitrils.

Die erfindungsgemässen Verbindungen werden in für derartige Substanzen üblicher Weise hergestellt, beispielsweise durch Umsetzung eines 4-Alkyl-2-hydroxyimethylencyclohexanons der Formel (II)

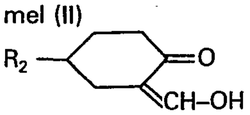

(II)

worin $R_2$ eine Alkylgruppe mit bis zu 10 Kohlenstoffatomen bedeutet, mit einem Benzamidin-hydrochlorid der Formel (III)

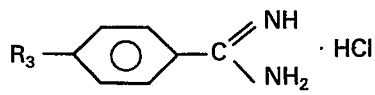

(III)

in Gegenwart eines basischen Kondensationsmittels. Der Rest $R_3$ hat darin die Bedeutung solcher für $R_1$ in Formel (I) angegebener Gruppen, die unter den Bedingungen derartiger Kondensationsreaktionen beständig sind, also insbesondere Alkyl, Alkoxy, Halogen oder $NO_2$. Darüberhinaus können als Rest $R_3$ auch andere unter diesen Bedingungen beständige Gruppen stehen, die sich leicht in andere Reste $R_1$ umwandeln lassen, insbesondere zum Beispiel die Benzyloxygruppe. Geeignete basische Kondensationsmittel für das erfindungsgemässe Verfahren sind beispielsweise Alkalimetallalkoholate oder Alkalimetallhydroxide in Alkoholen, aber auch organische Basen wie zum Beispiel Piperidin in Alkohol oder Pyridin. Bevorzugt werden Natriummethylat bzw. Natriumethylat in Methanol bzw. Ethanol verwendet. Die Reaktion wird bei einer Temperatur zwischen 20 °C und 120 °C durchgeführt, vorzugsweise in siedendem Methanol bzw. Ethanol. Unter diesen Bedingungen ist die Reaktion in der Regel nach 1–10 Stunden beendet. Die Aufarbeitung des Reaktionsgemisches erfolgt in an sich üblicher Weise, gewöhnlich durch Eindampfen, Aufnehmen mit Wasser und Extrahieren des gebildeten Tetrahydrochinazolins mit Ether.

Die sich gegebenenfalls anschliessende Umwandlung eines nicht gewünschten Restes $R_3$ in einen gewünschten Rest $R_1$ erfolgt unter für derartige Reaktionen üblichen Bedingungen. So werden beispielsweise die 2-(p-Benzyloxyphenyl)-tetrahydrochinazoline (Formel IV, $R_3$ = Benzyloxy) hydrolytisch oder hydrogenolytisch

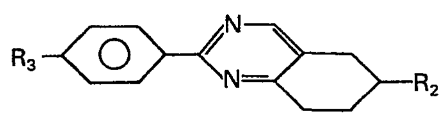

(IV)

zu den entsprechenden 2-(p-Hydroxyphenyl)-tetrahydrochinazolinen gespalten und anschliessend mit einem Alkanoylchlorid oder einem Alkoxycarbonylchlorid in Verbindungen der Formel (I) überführt, in denen $R_1$ Alkanoyloxy oder Alkoxycarbonyloxy bedeutet. Durch Umsetzung einer Verbindung der Formel (IV) in der $R_3$ Halogen, zum Beispiel Brom bedeutet, mit Kupfer-(I)-cyanid in N-Methylpyrrolidon wird das Halogenatom gegen die Nitrilgruppe ausgetauscht und so die 2-(p-Cyanophenyl)-tetrahydrochinazoline der Formel (I) hergestellt. Aus den Cyanoverbindungen wiederum werden durch Verseifung zu den entsprechenden Carbonsäuren und deren Umsetzung mit Schwefeltetrafluorid die erfindungsgemässen 2-(p-Trifluormethylphenyl)-tetrahydrochinazoline erhalten.

Die erfindungsgemässen flüssigkristallinen Dielektrika bestehen aus zwei oder mehr Komponenten, darunter mindestens eine der Formel (I). Die weiteren Komponenten sind vorzugsweise nematische oder nematogene Substanzen aus den Klassen der Azobenzole, Azoxybenzole, Biphenyle, Schiffschen Basen, insbesondere Benzyliden-Derivate, Phenylbenzoate, Phenylpyrimidine, Phenylcyclohexane, gegebenenfalls halogenierten Stilbene, Diphenylacetylen-Derivate, Diphenylnitrone und substituierten Zimtsäuren.

Die wichtigsten als derartige weitere Komponenten in Frage kommenden Verbindungen lassen sich durch die Formel (V) charakterisieren:

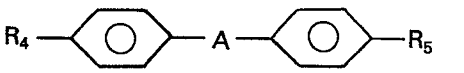 (V)

worin

A $-CH{=}CH-$ 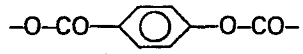

$-CX'{=}CH-$ 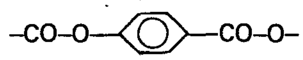

$-CH{=}CX'-$ 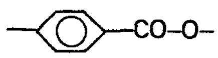

$-C{\equiv}C-$ 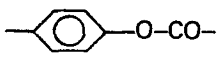

$-N{=}N-$ 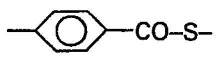

$-N(O){=}N-$ 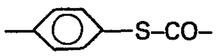

$-N{=}N(O)-$ $-CH{=}N-$
$-O-CO-$ $-N{=}CH-$
$-CO-O-$ $-CH{=}N(O)-$
$-S-CO-$ $-N(O){=}CH-$

$-CO-S-$ 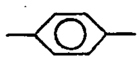 oder eine C–C-Einfachbindung

bedeutet. Weitere mögliche Komponenten der erfindungsgemässen Dielektrika sind solche Verbindungen der Formel (V), in denen einer oder mehrere Phenylringe durch eine entsprechende Zahl von trans-Cyclohexylringen ersetzt sind; ferner kann auch einer dieser Ringe ein 2,5-disubstituierter Pyrimidinring sein.

X' bedeutet Halogen, vorzugsweise Cl oder –CN; $R_5$ und $R_4$ sind gleich oder verschieden und können Alkyl-, Alkoxy-, Alkanoyl-, Alkanoyloxy- oder Alkoxycarbonyloxyreste mit bis zu 18, vorzugsweise bis zu 8 C-Atomen bedeuten; weiterhin kann auch einer dieser Reste –CN, –NC, $NO_2$, $CF_3$ oder Halogen bedeuten.

Bei den meisten dieser Verbindungen sind $R_5$ und $R_4$ vorzugsweise verschieden, wobei einer der Reste meist eine Alkyl- oder Alkoxygruppe bedeutet. Aber auch eine grosse Zahl anderer Varianten der vorgesehenen Substituenten sind gebräuchlich. Viele solcher Substanzen sind im Handel erhältlich.

Die erfindungsgemässen Dielektrika enthalten in der Regel mindestens 30, vorzugsweise 50–99, insbesondere 60–98 Gewichtsteile der Verbindungen der Formel (I) und (V). Hiervon entfallen bevorzugt 5–50 Gewichtsteile, insbesondere 10–30 Gewichtsteile auf eine oder mehrere Verbindungen der Formel (I). Es werden von der Erfindung auch solche flüssigkristallinen Dielektrika umfasst, denen beispielsweise zu Dotierungszwecken nur weniger als 5 Gewichtsteile, zum Beispiel 0,1 bis 3 Gewichtsteile einer oder mehrerer Verbindungen der Formel (I) zugesetzt worden sind.

Die Herstellung der erfindungsgemässen Dielektrika erfolgt in an sich üblicher Weise. In der Regel wird die gewünschte Menge der in geringerer Menge verwendeten Komponenten in den den Hauptbestandteil ausmachenden Komponenten gelöst, zweckmässig bei erhöhter Temperatur. Wenn dabei eine Temperatur oberhalb des Klärpunkts des Hauptbestandteils gewählt wird, kann die Vollständigkeit des Lösevorgangs besonders leicht beobachtet werden.

Es ist jedoch auch möglich, Lösungen der Komponenten der Formel (I) und (V) in einem geeigneten organischen Lösungsmittel, zum Beispiel Aceton, Chloroform oder Methanol, zu mischen und das Lösungsmittel nach gründlicher Durchmischung wieder zu entfernen, beispielsweise durch Destillation unter vermindertem Druck. Selbstverständlich muss bei dieser Verfahrensweise darauf geachtet werden, dass durch das Lösungsmittel keine Verunreinigungen oder unerwünschten Dotierungsstoffe eingeschleppt werden.

Durch geeignete Zusätze können die flüssigkristallinen Dielektrika nach der Erfindung so modifiziert werden, dass sie in allen bisher bekannt gewordenen Arten von Flüssigkristallanzeigeelementen verwendet werden können. Derartige Zusätze sind dem Fachmann bekannt und sind in der einschlägigen Literatur ausführlich beschrieben. Beispielsweise können Substanzen zur Veränderung der Viskosität, der Leitfähigkeit und/oder der Orientierung der nematischen Phasen zugesetzt werden. Derartige Substanzen sind zum Beispiel in den DE-OS 22 09 127, 23 38 281, 24 50 088, 25 48 360 und 26 37 430 beschrieben.

Die folgenden Beispiele sollen die Erfindung erläutern, ohne sie zu begrenzen. In den Beispielen bedeuten F. den Schmelzpunkt und K. den Klärpunkt einer flüssigkristallinen Substanz in Grad Celsius; Siedetemperaturen sind mit Kp. bezeichnet. Wenn nichts anderes angegeben ist, bedeuten Angaben von Teilen oder Prozent Gewichtsteile bzw. Gewichtsprozent.

Beispiel 1

Zu einer Lösung von 0,3 g Natrium in 14 ml Ethanol werden nacheinander 2,46 g 4-n-Hexyl-2-hydroxymethylencyclohexanon und 3,1 g 4-n-Butyloxy-benzamidin-hydrochlorid gegeben und das Reaktionsgemisch 4 Stunden unter Rühren zum Sieden erhitzt. Anschliessend wird das Ethanol abdestilliert, der Rückstand mit 50 ml Wasser versetzt und das wässrige Reaktionsgemisch zweimal mit je 50 ml Diethylether extrahiert. Die Extrakte werden über Natriumsulfat getrocknet und eingedampft. Das zurückbleibende 2-(4-n-Butyloxyphenyl)-6-n-hexyl-5,6,7,8-tetrahydrochinazolin wird aus Ethanol umkristallisiert; Ausbeute 2,2 g, F. 73°, K. 105°.

Analog werden hergestellt:

2-(4-Methylphenyl)-6-methyl-5,6,7,8-tetrahydrochinazolin,
2-(4-Methylphenyl)-6-ethyl-5,6,7,8-tetrahydrochinazolin,
2-(4-Methylphenyl)-6-n-propyl-5,6,7,8-tetrahydrochinazolin,
2-(4-Methylphenyl)-6-n-butyl-5,6,7,8-tetrahydrochinazolin,
2-(4-Methylphenyl)-6-n-pentyl-5,6,7,8-tetrahydrochinazolin,
2-(4-Methylphenyl)-6-n-hexyl-5,6,7,8-tetrahydrochinazolin,
2-(4-Methylphenyl)-6-n-heptyl-5,6,7,8-tetrahydrochinazolin,
2-(4-Methylphenyl)-6-n-octyl-5,6,7,8-tetrahydrochinazolin,
2-(4-Methylphenyl)-6-n-nonyl-5,6,7,8-tetrahydrochinazolin,
2-(4-Methylphenyl)-6-n-decyl-5,6,7,8-tetrahydrochinazolin,
2-(4-Methylphenyl)-6-(2-methylbutyl)-5,6,7,8-tetrahydrochinazolin,
2-(4-Methylphenyl)-6-(2-ethylhexyl)-5,6,7,8-tetrahydrochinazolin

2-(4-Ethylphenyl)-6-methyl-5,6,7,8-tetrahydrochinazolin,
2-(4-Ethylphenyl)-6-ethyl-5,6,7,8-tetrahydrochinazolin,
2-(4-Ethylphenyl)-6-n-propyl-5,6,7,8-tetrahydrochinazolin,
2-(4-Ethylphenyl)-6-n-butyl-5,6,7,8-tetrahydrochinazolin,
2-(4-Ethylphenyl)-6-n-pentyl-5,6,7,8-tetrahydrochinazolin,
2-(4-Ethylphenyl)-6-n-hexyl-5,6,7,8-tetrahydrochinazolin,
2-(4-Ethylphenyl)-6-n-heptyl-5,6,7,8-tetrahydrochinazolin,
2-(4-Ethylphenyl)-6-n-octyl-5,6,7,8-tetrahydrochinazolin,
2-(4-Ethylphenyl)-6-n-nonyl-5,6,7,8-tetrahydrochinazolin,
2-(4-Ethylphenyl)-6-n-decyl-5,6,7,8-tetrahydrochinazolin,
2-(4-Ethylphenyl)-6-(2-methylbutyl)-5,6,7,8-tetrahydrochinazolin,
2-(4-Ethylphenyl)-6-(2-ethylhexyl)-5,6,7,8-tetrahydrochinazolin,

2-(4-n-Propylphenyl)-6-methyl-5,6,7,8-tetrahydrochinazolin,
2-(4-n-Propylphenyl)-6-ethyl-5,6,7,8-tetrahydrochinazolin,
2-(4-n-Propylphenyl)-6-n-propyl-5,6,7,8-tetrahydrochinazolin,
2-(4-n-Propylphenyl)-6-n-butyl-5,6,7,8-tetrahydrochinazolin,
2-(4-n-Propylphenyl)-6-n-pentyl-5,6,7,8-tetrahydrochinazolin,
2-(4-n-Propylphenyl)-6-n-hexyl-5,6,7,8-tetrahydrochinazolin,
2-(4-n-Propylphenyl)-6-n-heptyl-5,6,7,8-tetrahydrochinazolin,
2-(4-n-Propylphenyl)-6-n-octyl-5,6,7,8-tetrahydrochinazolin,
2-(4-n-Propylphenyl)-6-n-nonyl-5,6,7,8-tetrahydrochinazolin,
2-(4-n-Propylphenyl)-6-n-decyl-5,6,7,8-tetrahydrochinazolin,
2-(4-n-Propylphenyl)-6-(2-methylbutyl)-5,6,7,8-tetrahydrochinazolin,
2-(4-n-Propylphenyl)-6- (2-ethylhexyl)-5,6,7,8-tetrahydrochinazolin,

2-(4-n-Butylphenyl)-6-methyl-5,6,7,8-tetrahydrochinazolin,
2-(4-n-Butylphenyl)-6-methyl-5,6,7,8-tetrahydrochinazolin,
2-(4-n-Butylphenyl)-6-n-propyl-5,6,7,8-tetrahydrochinazolin,
2-(4-n-Butylphenyl)-6-n-butyl-5,6,7,8-tetrahydrochinazolin,
2-(4-n-Butylphenyl)-6-n-pentyl-5,6,7,8-tetrahydrochinazolin,
2-(4-n-Butylphenyl)-6-n-hexyl-5,6,7,8-tetrahydrochinazolin,
2-(4-n-Butylphenyl)-6-n-heptyl-5,6,7,8-tetrahydrochinazolin,
2-(4-n-Butylphenyl)-6-n-octyl-5,6,7,8-tetrahydrochinazolin,
2-(4-n-Butylphenyl)-6-n-nonyl-5,6,7,8-tetrahydrochinazolin,
2-(4-n-Butylphenyl)-6-n-decyl-5,6,7,8-tetrahydrochinazolin,
2-(4-n-Butylphenyl)-6-(2-methylbutyl)-5,6,7,8-tetrahydrochinazolin,
2-(4-n-Butylphenyl)-6-(2-ethylhexyl)-5,6,7,8-tetrahydrochinazolin,

2-(4-n-Pentylphenyl)-6-methyl-5,6,7,8-tetrahydrochinazolin,
2-(4-n-Pentylphenyl)-6-ethyl-5,6,7,8-tetrahydrochinazolin,
2-(4-n-Pentylphenyl)-6-n-propyl-5,6,7,8-tetrahydrochinazolin,
2-(4-n-Pentylphenyl)-6-n-butyl-5,6,7,8-tetrahydrochinazolin,
2-(4-n-Pentylphenyl)-6-n-pentyl-5,6,7,8-tetrahydrochinazolin,
2-(4-n-Penylyphenyl)-6-n-hexyl-5,6,7,8-tetrahydrochinazolin
2-(4-n-Pentylphenyl)-6-n-heptyl-5,6,7,8-tetrahydrochinazolin,
2-(4-n-Pentylphenyl)-6-n-octyl-5,6,7,8-tetrahydrochinazolin,
2-(4-n-Pentylphenyl)-6-n-nonyl-5,6,7,8-tetrahydrochinazolin,
2-(4-n-Pentylphenyl)-6-n-decyl-5,6,7,8-tetrahydrochinazolin,
2-(4-n-Pentylphenyl)-6-(2-methylbutyl)-5,6,7,8-tetrahydrochinazolin,
2-(4-n-Pentylphenyl)-6-(2-ethylhexyl)-5,6,7,8-tetrahydrochinazolin,

2-(4-n-Hexylphenyl)-6-methyl-5,6,7,8-tetrahydrochinazolin,
2-(4-n-Hexylphenyl)-6-ethyl-5,6,7,8-tetrahydrochinazolin,

2-(4-n-Hexylphenyl)-6-n-propyl-5,6,7,8-tetrahydrochinazolin,
2-(4-n-Hexylphenyl)-6-n-butyl-5,6,7,8-tetrahydrochinazolin,
2-(4-n-Hexylphenyl)-6-n-pentyl-5,6,7,8-tetrahydrochinazolin,
2-(4-n-Hexylphenyl)-6-n-hexyl-5,6,7,8-tetrahydrochinazolin,
2-(4-n-Hexylphenyl)-6-n-heptyl-5,6,7,8-tetrahydrochinazolin,
2-(4-n-Hexylphenyl)-6-n-octyl-5,6,7,8-tetrahydrochinazolin,
2-(4-n-Hexylphenyl)-6-n-nonyl-5,6,7,8-tetrahydrochinazolin,
2-(4-n-Hexylphenyl)-6-n-decyl-5,6,7,8-tetrahydrochinazolin,
2-(4-n-Hexylphenyl)-6-(2-methylbutyl)-5,6,7,8-tetrahydrochinazolin,
2-(4-n-Hexylphenyl)-6-(2-ethylhexyl)-5,6,7,8-tetrahydrochinazolin,

2-(4-n-Heptylphenyl)-6-methyl-5,6,7,8-tetrahydrochinazolin,
2-(4-n-Heptylphenyl)-6-ethyl-5,6,7,8-tetrahydrochinazolin,
2-(4-n-Heptylphenyl)-6-n-propyl-5,6,7,8-tetrahydrochinazolin,
2-(4-n-Heptylphenyl)-6-n-butyl-5,6,7,8-tetrahydrochinazolin,
2-(4-n-Heptylphenyl)-6-n-pentyl-5,6,7,8-tetrahydrochinazolin,
2-(4-n-Heptylphenyl)-6-n-hexyl-5,6,7,8-tetrahydrochinazolin,
2-(4-n-Heptylphenyl)-6-n-heptyl-5,6,7,8-tetrahydrochinazolin,
2-(4-n-Heptylphenyl)-6-n-octyl-5,6,7,8-tetrahydrochinazolin,
2-(4-n-Heptylphenyl)-6-n-nonyl-5,6,7,8-tetrahydrochinazolin,
2-(4-n-Heptylphenyl)-6-(2-methylbutyl)-5,6,7,8-tetrahydrochinazolin,
2-(4-n-Heptylphenyl)-6-(2-ethylhexyl)-5,6,7,8-tetrahydrochinazolin,

2-(4-n-Octylphenyl)-6-methyl-5,6,7,8-tetrahydrochinazolin,
2-(4-n-Octylphenyl)-6-ethyl-5,6,7,8-tetrahydrochinazolin,
2-(4-n-Octylphenyl)-6-n-propyl-5,6,7,8-tetrahydrochinazolin,
2-(4-n-Octylphenyl)-6-n-butyl-5,6,7,8-tetrahydrochinazolin,
2-(4-n-Octylphenyl)-6-n-pentyl-5,6,7,8-tetrahydrochinazolin,
2-(4-n-Octylphenyl)-6-n-hexyl-5,6,7,8-tetrahydrochinazolin,
2-(4-n-Octylphenyl)-6-n-heptyl-5,6,7,8-tetrahydrochinazolin,
2-(4-n-Octylphenyl)-6-n-octyl-5,6,7,8-tetrahydrochinazolin,
2-(4-n-Octylphenyl)-6-(2-methylbutyl)-5,6,7,8-tetrahydrochinazolin,
2-(4-n-Octylphenyl)-6-(2-ethylhexyl)-5,6,7,8-tetrahydrochinazolin,

2-[4-(2-Methylbutyl)-phenyl]-6-methyl-5,6,7,8-tetrahydrochinazolin,
2-[4-(2-Methylbutyl)-phenyl]-6-ethyl-5,6,7,8-tetrahydrochinazolin,
2-[4-(2-Methylbutyl)-phenyl]-6-n-propyl-5,6,7,8-tetrahydrochinazolin,
2-[4-(2-Methylbutyl)-phenyl]-6-n-butyl-5,6,7,8-tetrahydrochinazolin,
2-[4-(2-Methylbutyl)-phenyl]-6-n-pentyl-5,6,7,8-tetrahydrochinazolin,
2-[4-(2-Methylbutyl)-phenyl]-6-n-hexyl-5,6,7,8-tetrahydrochinazolin,
2-[4-(2-Methylbutyl)-phenyl]-6-n-heptyl-5,6,7,8-tetrahydrochinazolin,
2-[4-(2-Methylbutyl)-phenyl]-6-n-octyl-5,6,7,8-tetrahydrochinazolin,
2-[4-(2-Methylbutyl)-phenyl]-6-n-nonyl-5,6,7,8-tetrahydrochinazolin,
2-[4-(2-Methylbutyl)-phenyl]-6-n-decyl-5,6,7,8-tetrahydrochinazolin,

2-[4-(1-Methylheptyl)-phenyl]-6-methyl-5,6,7,8-tetrahydrochinazolin,
2-[4-(1-Methylheptyl)-phenyl]-6-ethyl-5,6,7,8-tetrahydrochinazolin,
2-[4-(1-Methylheptyl)-phenyl]-6-n-propyl-5,6,7,8-tetrahydrochinazolin,
2-[4-(1-Methylheptyl)-phenyl]-6-n-butyl-5,6,7,8-tetrahydrochinazolin,
2-[4-(1-Methylheptyl)-phenyl]-6-n-pentyl-5,6,7,8-tetrahydrochinazolin,
2-[4-(1-Methylheptyl)-phenyl]-6-n-hexyl-5,6,7,8-tetrahydrochinazolin,
2-[4-(1-Methylheptyl)-phenyl]-6-n-heptyl-5,6,7,8-tetrahydrochinazolin,
2-[4-(1-Methylheptyl)-phenyl]-6-n-octyl-5,6,7,8-tetrahydrochinazolin,
2-[4-(1-Methylheptyl)-phenyl]-6-n-nonyl-5,6,7,8-tetrahydrochinazolin,
2-[4-(1-Methylheptyl)-phenyl]-6-n-decyl-5,6,7,8-tetrahydrochinazolin,

2-(4-Methoxyphenyl)-6-methyl-5,6,7,8-tetrahydrochinazolin,
2-(4-Methoxyphenyl)-6-ethyl-5,6,7,8-tetrahydrochinazolin,
2-(4-Methoxyphenyl)-6-n-propyl-5,6,7,8-tetrahydrochinazolin,
2-(4-Methoxyphenyl)-6-n-butyl-5,6,7,8-tetrahydrochinazolin,
2-(4-Methoxyphenyl)-6-n-pentyl-5,6,7,8-tetrahydrochinazolin,
2-(4-Methoxyphenyl)-6-n-hexyl-5,6,7,8-tetrahydrochinazolin,
2-(4-Methoxyphenyl)-6-n-heptyl-5,6,7,8-tetrahydrochinazolin,
2-(4-Methoxyphenyl)-6-n-octyl-5,6,7,8-tetrahydrochinazolin,
2-(4-Methoxyphenyl)-6-n-nonyl-5,6,7,8-tetrahydrochinazolin,
2-(4-Methoxyphenyl)-6-n-decyl-5,6,7,8-tetrahydrochinazolin,
2-(4-Methoxyphenyl)-6-(2-methylbutyl)-5,6,7,8-tetrahydrochinazolin,

2-(4-Methoxyphenyl)-6-(2-ethylhexyl)-5,6,7,8-tetrahydrochinazolin,

2-(4-Ethoxyphenyl)-6-methyl-5,6,7,8-tetrahydrochinazolin,
2-(4-Ethoxyphenyl)-6-ethyl-5,6,7,8-tetrahydrochinazolin,
2-(4-Ethoxyphenyl)-6-n-propyl-5,6,7,8-tetrahydrochinazolin,
2-(4-Ethoxyphenyl)-6-n-butyl-5,6,7,8-tetrahydrochinazolin,
2-(4-Ethoxyphenyl)-6-n-hexyl-5,6,7,8-tetrahydrochinazolin,
2-(4-Ethoxyphenyl)-6-n-heptyl-5,6,7,8-tetrahydrochinazolin,
2-(4-Ethoxyphenyl)-6-n-octyl-5,6,7,8-tetrahydrochinazolin,
2-(4-Ethoxyphenyl)-6-n-nonyl-5,6,7,8-tetrahydrochinazolin,
2-(4-Ethoxyphenyl)-6-n-decyl-5,6,7,8-tetrahydrochinazolin,
2-(4-Ethoxyphenyl)-6-(2-methylbutyl)-5,6,7,8-tetrahydrochinazolin,
2-(4-Ethoxyphenyl)-6-(2-ethylhexyl)-5,6,7,8-tetrahydrochinazolin,

2-(4-n-Propyloxyphenyl)-6-methyl-5,6,7,8-tetrahydrochinazolin,
2-(4-n-Propyloxyphenyl)-6-ethyl-5,6,7,8-tetrahydrochinazolin,
2-(4-n-Propyloxyphenyl)-6-n-propyl-5,6,7,8-tetrahydrochinazolin,
2-(4-n-Propyloxyphenyl)-6-n-butyl-5,6,7,8-tetrahydrochinazolin,
2-(4-n-Propyloxyphenyl)-6-n-pentyl-5,6,7,8-tetrahydrochinazolin,
2-(4-n-Propyloxyphenyl)-6-n-hexyl-5,6,7,8-tetrahydrochinazolin,
2-(4-n-Propyloxyphenyl)-6-n-heptyl-5,6,7,8-tetrahydrochinazolin,
2-(4-n-Propyloxyphenyl)-6-n-octyl-5,6,7,8-tetrahydrochinazolin,
2-(4-n-Propyloxyphenyl)-6-n-nonyl-5,6,7,8-tetrahydrochinazolin,
2-(4-Propyloxyphenyl)-6-n-decyl-5,6,7,8-tetrahydrochinazolin,
2-(4-n-Propyloxyphenyl)-6-(2-methylbutyl)-5,6,7,8-tetrahydrochinazolin,
2-(4-n-Propyloxyphenyl)-6-(2-ethylhexyl)-5,6,7,8-tetrahydrochinazolin,

2-(4-n-Butyloxyphenyl)-6-methyl-5,6,7,8-tetrahydrochinazolin,
2-(4-n-Butyloxyphenyl)-6-ethyl-5,6,7,8-tetrahydrochinazolin,
2-(4-n-Butyloxyphenyl)-6-n-propyl-5,6,7,8-tetrahydrochinazolin,
2-(4-n-Butyloxyphenyl)-6-n-butyl-5,6,7,8-tetrahydrochinazolin,
2-(4-n-Butyloxyphenyl)-6-n-pentyl-5,6,7,8-tetrahydrochinazolin,
2-(4-n-Butyloxyphenyl)-6-n-heptyl-5,6,7,8-tetrahydrochinazolin,
2-(4-n-Butyloxyphenyl)-6-n-octyl-5,6,7,8-tetrahydrochinazolin,
2-(4-n-Butyloxyphenyl)-6-n-nonyl-5,6,7,8-tetrahydrochinazolin,
2-(4-n-Butyloxyphenyl)-6-n-decyl-5,6,7,8-tetrahydrochinazolin,
2-(4-n-Butyloxyphenyl)-6-(2-methylbutyl)-5,6,7,8-tetrahydrochinazolin,
2-(4-n-Butyloxyphenyl)-6-(2-ethylhexyl)-5,6,7,8-tetrahydrochinazolin,

2-(4-n-Pentyloxyphenyl)-6-methyl-5,6,7,8-tetrahydrochinazolin,
2-(4-n-Pentyloxyphenyl)-6-ethyl-5,6,7,8-tetrahydrochinazolin,
2-(4-n-Pentyloxyphenyl)-6-n-propyl-5,6,7,8-tetrahydrochinazolin,
2-(4-n-Pentyloxyphenyl)-6-n-butyl-5,6,7,8-tetrahydrochinazolin,
2-(4-Pentyloxyphenyl)-6-n-pentyl-5,6,7,8-tetrahydrochinazolin,
2-(4-n-Pentyloxyphenyl)-6-n-hexyl-5,6,7,8-tetrahydrochinazolin,
2-(4-n-Pentyloxyphenyl)-6-n-heptyl-5,6,7,8-tetrahydrochinazolin,
2-(4-n-Pentyloxyphenyl)-6-n-octyl-5,6,7,8-tetrahydrochinazolin,
2-(4-n-Pentyloxyphenyl)-6-n-nonyl-5,6,7,8-tetrahydrochinazolin,
2-(4-n-Pentyloxyphenyl)-6-n-decyl-5,6,7,8-tetrahydrochinazolin,
2-(4-n-Pentyloxyphenyl)-6-(2-methylbutyl)-5,6,7,8-tetrahydrochinazolin,
2-(4-n-Pentyloxyphenyl)-6-(2-ethyloxyphenyl)-5,6,7,8-tetrahydrochinazolin,

2-(4-n-Hexyloxyphenyl)-6-methyl-5,6,7,8-tetrahydrochinazolin,
2-(4-n-Hexyloxyphenyl)-6-ethyl-5,6,7,8-tetrahydrochinazolin,
2-(4-n-Hexyloxyphenyl)-6-n-propyl-5,6,7,8-tetrahydrochinazolin,
2-(4-n-Hexyloxyphenyl)-6-n-butyl-5,6,7,8-tetrahydrochinazolin,
2-(4-n-Hexyloxyphenyl)-6-n-pentyl-5,6,7,8-tetrahydrochinazolin,
2-(4-n-Hexyloxyphenyl)-6-n-hexyl-5,6,7,8-tetrahydrochinazolin,
2-(4-n-Hexyloxyphenyl)-6-n-heptyl-5,6,7,8-tetrahydrochinazolin,
2-(4-n-Hexyloxyphenyl)-6-n-octyl-5,6,7,8-tetrahydrochinazolin,
2-(4-n-Hexyloxyphenyl)-6-n-nonyl-5,6,7,8-tetrahydrochinazolin,
2-(4-n-Hexyloxyphenyl)-6-n-decyl-5,6,7,8-tetrahydrochinazolin,
2-(4-n-Hexyloxyphenyl)-6-(2-methylbutyl)-5,6,7,8-tetrahydrochinazolin,
2-(4-n-Hexyloxyphenyl)-6-(2-ethylhexyl)-5,6,7,8-tetrahydrochinazolin,

2-(4-n-Heptyloxyphenyl)-6-methyl-5,6,7,8-tetrahydrochinazolin,
2-(4-n-Heptyloxyphenyl)-6-ethyl-5,6,7,8-tetrahydrochinazolin,
2-(4-n-Heptyloxyphenyl)-6-n-propyl-5,6,7,8-tetrahydrochinazolin,

2-(4-n-Heptyloxyphenyl)-6-n-butyl-5,6,7,8-tetrahydrochinazolin,
2-(4-n-Heptyloxyphenyl)-6-n-pentyl-5,6,7,8-tetrahydrochinazolin,
2-(4-n-Heptyloxyphenyl)-6-n-hexyl-5,6,7,8-tetrahydrochinazolin,
2-(4-n-Heptyloxyphenyl)-6-n-heptyl-5,6,7,8-tetrahydrochinazolin,
2-(4-n-Heptyloxyphenyl)-6-n-octyl-5,6,7,8-tetrahydrochinazolin,
2-(4-n-Heptyloxyphenyl)-6-n-nonyl-5,6,7,8-tetrahydrochinazolin,
2-(4-n-Heptyloxyphenyl)-6-(2-methylbutyl)-5,6,7,8-tetrahydrochinazolin,
2-(4-n-Heptyloxyphenyl)-6-(2-ethylhexyl)-5,6,7,8-tetrahydrochinazolin,

2-(4-n-Octyloxyphenyl)-6-methyl-5,6,7,8-tetrahydrochinazolin,
2-(4-n-Octyloxyphenyl)-6-ethyl-5,6,7,8-tetrahydrochinazolin,
2-(4-n-Octyloxyphenyl)-6-n-propyl-5,6,7,8-tetrahydrochinazolin,
2-(4-n-Octyloxyphenyl)-6-n-butyl-5,6,7,8-tetrahydrochinazolin,
2-(4-n-Octyloxyphenyl)-6-n-pentyl-5,6,7,8-tetrahydrochinazolin,
2-(4-n-Octyloxyphenyl)-6-n-hexyl-5,6,7,8-tetrahydrochinazolin,
2-(4-n-Octyloxyphenyl)-6-n-heptyl-5,6,7,8-tetrahydrochinazolin,
2-(4-n-Octyloxyphenyl)-6-n-octyl-5,6,7,8-tetrahydrochinazolin,
2-(4-n-Octyloxyphenyl)-6-(2-methylbutyl)-5,6,7,8-tetrahydrochinazolin,
2-(4-n-Octyloxyphenyl)-6-(2-ethylhexyl)-5,6,7,8-tetrahydrochinazolin,

2-[4-(2-Ethylhexyloxy)phenyl]-6-methyl-5,6,7,8-tetrahydrochinazolin,
2-[4-(2-Ethylhexyloxy)phenyl]-6-ethyl-5,6,7,8-tetrahydrochinazolin,
2-[4-(2-Ethylhexyloxy)phenyl]-6-n-propyl-5,6,7,8-tetrahydrochinazolin,
2-[4-(2-Ethylhexyloxy)phenyl]-6-n-butyl-5,6,7,8-tetrahydrochinazolin,
2-[4-(2-Ethylhexyloxy)phenyl]-6-n-pentyl-5,6,7,8-tetrahydrochinazolin,
2-[4-(2-Ethylhexyloxy)phenyl]-6-n-hexyl-5,6,7,8-tetrahydrochinazolin,
2-[4-(2-Ethylhexyloxy)phenyl]-6-n-heptyl-5,6,7,8-tetrahydrochinazolin,
2-[4-(2-Ethylhexyloxy)phenyl]-6-n-octyl-5,6,7,8-tetrahydrochinazolin,
2-[4-(2-Ethylhexyloxy)phenyl]-6-n-nonyl-5,6,7,8-tetrahydrochinazolin,

2-(4-Chlorphenyl)-6-methyl-5,6,7,8-tetrahydrochinazolin,
2-(4-Chlorphenyl)-6-ethyl-5,6,7,8-tetrahydrochinazolin,
2-(4-Chlorphenyl)-6-n-propyl-5,6,7,8-tetrahydrochinazolin,
2-(4-Chlorphenyl)-6-n-butyl-5,6,7,8-tetrahydrochinazolin,
2-(4-Chlorphenyl)-6-n-pentyl-5,6,7,8-tetrahydrochinazolin,
2-(4-Chlorphenyl)-6-n-hexyl-5,6,7,8-tetrahydrochinazolin,
2-(4-Chlorphenyl)-6-n-heptyl-5,6,7,8-tetrahydrochinazolin,
2-(4-Chlorphenyl)-6-n-octyl-5,6,7,8-tetrahydrochinazolin,
2-(4-Chlorphenyl)-6-n-nonyl-5,6,7,8-tetrahydrochinazolin,
2-(4-Chlorphenyl)-6-n-decyl-5,6,7,8-tetrahydrochinazolin,
2-(4-Chlorphenyl)-6-(2-methylbutyl)-5,6,7,8-tetrahydrochinazolin,
2-(4-Chlorphenyl)-6-(2-ethylhexyl)-5,6,7,8-tetrahydrochinazolin,

2-(4-Bromphenyl)-6-methyl-5,6,7,8-tetrahydrochinazolin,
2-(4-Bromphenyl)-6-ethyl-5,6,7,8-tetrahydrochinazolin,
2-(4-Bromphenyl)-6-n-propyl-5,6,7,8-tetrahydrochinazolin,
2-(4-Bromphenyl)-6-n-butyl-5,6,7,8-tetrahydrochinazolin,
2-(4-Bromphenyl)-6-n-pentyl-5,6,7,8-tetrahydrochinazolin,
2-(4-Bromphenyl)-6-n-hexyl-5,6,7,8-tetrahydrochinazolin, F. 167°;
2-(4-Bromphenyl)-6-n-heptyl-5,6,7,8-tetrahydrochinazolin,
2-(4-Bromphenyl)-6-n-octyl-5,6,7,8-tetrahydrochinazolin ,
2-(4-Bromphenyl)-6-n-nonyl-5,6,7,8-tetrahydrochinazolin,
2-(4-Bromphenyl)-6-n-decyl-5,6,7,8-tetrahydrochinazolin,
2-(4-Bromphenyl)-6-(2-methylbutyl)-5,6,7,8-tetrahydrochinazolin,

2-(4-Fluorphenyl)-6-methyl-5,6,7,8-tetrahydrochinazolin,
2-(4-Fluorphenyl)-6-ethyl-5,6,7,8-tetrahydrochinazolin,
2-(4-Fluorphenyl)-6-n-propyl-5,6,7,8-tetrahydrochinazolin,
2-(4-Fluorphenyl)-6-n-butyl-5,6,7,8-tetrahydrochinazolin,
2-(4-Fluorphenyl)-6-n-pentyl-5,6,7,8-tetrahydrochinazolin,
2-(4-Fluorphenyl)-6-n-hexyl-5,6,7,8-tetrahydrochinazolin,
2-(4-Fluorphenyl)-6-n-heptyl-5,6,7,8-tetrahydrochinazolin,
2-(4-Fluorphenyl)-6-n-octyl-5,6,7,8-tetrahydrochinazolin,
2-(4-Fluorphenyl)-6-n-nonyl-5,6,7,8-tetrahydrochinazolin,
2-(4-Fluorphenyl)-6-n-decyl-5,6,7,8-tetrahydrochinazolin,
2-(4-Fluorphenyl)-6-(2-methylbutyl)-5,6,7,8-tetrahydrochinazolin,
2-(4-Fluorphenyl)-6-(2-ethylhexyl)-5,6,7,8-tetrahydrochinazolin,

2-(4-Nitrophenyl)-6-methyl-5,6,7,8-tetrahydrochinazolin,
2-(4-Nitrophenyl)-6-ethyl-5,6,7,8-tetrahydrochinazolin,
2-(4-Nitrophenyl)-6-n-propyl-5,6,7,8-tetrahydrochinazolin,
2-(4-Nitrophenyl)-6-n-butyl-5,6,7,8-tetrahydrochinazolin,
2-(4-Nitrophenyl)-6-n-pentyl-5,6,7,8-tetrahydrochinazolin,
2-(4-Nitrophenyl)-6-n-hexyl-5,6,7,8-tetrahydrochinazolin,
2-(4-Nitrophenyl)-6-n-heptyl-5,6,7,8-tetrahydrochinazolin,
2-(4-Nitrophenyl)-6-n-octyl-5,6,7,8-tetrahydrochinazolin,
2-(4-Nitrophenyl)-6-n-nonyl-5,6,7,8-tetrahydrochinazolin,
2-(4-Nitrophenyl)-6-n-decyl-5,6,7,8-tetrahydrochinazolin,
2-(4-Nitrophenyl)-6-(2-methylbutyl)-5,6,7,8-tetrahydrochinazolin,
2-(4-Nitrophenyl)-6-(2-ethylhexyl)-5,6,7,8-tetrahydrochinazolin.

Beispiel 2

26,5 g 2-(4-Bromphenyl)-6-n-hexyl-5,6,7,8-tetrahydrochinazolin und 7,6 g Kupfer-(I)-cyanid werden in 70 ml N-Methyl pyrrolidon-(2) unter Rühren 2,5 Stunden auf 200° erwärmt. Nach dem Abkühlen wird das Reaktionsgemisch in 300 ml Eiswasser gegossen, der sich dabei bildende Niederschlag abfiltriert und mit Wasser gewaschen. Nach dem Trocknen wird der schwarzgraue Niederschlag fünfmal mit je 50 ml Diethylether digeriert, die Etherfiltrate über Natriumsulfat getrocknet und eingedampft. Das zurückbleibende 2-(4-Cyanophenyl)-6-n-hexyl-5,6,7,8-tetrahydrochinazolin wird unter Zusatz von Aktivkohle aus Ethanol umkristallisiert; Ausbeute 17,3 g farblose Kristalle, F. 90°, K. 108°.

Analog werden hergestellt:
2-(4-Cyanophenyl)-6-methyl-5,6,7,8-tetrahydrochinazolin,
2-(4-Cyanophenyl)-6-ethyl-5,6,7,8-tetrahydrochinazolin,
2-(4-Cyanophenyl)-6-n-propyl-5,6,7,8-tetrahydrochinazolin,
2-(4-Cyanophenyl)-6-n-butyl-5,6,7,8-tetrahydrochinazolin,
2-(4-Cyanophenyl)-6-n-pentyl-5,6,7,8-tetrahydrochinazolin, F. 98°, K. 114°,
2-(4-Cyanophenyl)-6-n-heptyl-5,6,7,8-tetrahydrochinazolin,
2-(4-Cyanophenyl)-6-n-octyl-5,6,7,8-tetrahydrochinazolin,
2-(4-Cyanophenyl)-6-n-nonyl-5,6,7,8-tetrahydrochinazolin,
2-(4-Cyanophenyl)-6-n-decyl-5,6,7,8-tetrahydrochinazolin,
2-(4-Cyanophenyl)-6-(2-methylbutyl)-5,6,7,8-tetrahydrochinazolin,
2-(4-Cyanophenyl)-6-(2-ethylhexyl)-5,6,7,8-tetrahydrochinazolin.

Beispiel 3

(a) 37,3 g 2-(4-Benzyloxyphenyl)-6-n-butyl-5,6,7,8-tetrahydrochinazolin werden in 300 ml Methanol bei 60° unter Normaldruck in Gegenwart von 10 g Palladium/Aktivkohle (5% Pd) bis zum Ende der Wasserstoffaufnahme hydriert. Anschliessend wird der Katalysator abfiltriert, das Filtrat eingedampft und das zurückbleibende 2-(4-Hydroxyphenyl)-6-n-butyl-5,6,7,8-tetrahydrochinazolin aus Ethanol um kristallisiert; Ausbeute 21,6 g.

Analog werden hergestellt:
2-(4-Hydroxyphenyl)-6-methyl-5,6,7,8-tetrahydrochinazolin,
2-(4-Hydroxyphenyl)-6-ethyl-5,6,7,8-tetrahydrochinazolin,
2-(4-Hydroxyphenyl)-6-n-propyl-5,6,7,8-tetrahydrochinazolin,
2-(4-Hydroxyphenyl)-6-n-pentyl-5,6,7,8-tetrahydrochinazolin,
2-(4-Hydroxyphenyl)-6-n-hexyl-5,6,7,8-tetrahydrochinazolin,
2-(4-Hydroxyphenyl)-6-n-heptyl-5,6,7,8-tetrahydrochinazolin,
2-(4-Hydroxyphenyl)-6-n-octyl-5,6,7,8-tetrahydrochinazolin,
2-(4-Hydroxyphenyl)-6-n-nonyl-5,6,7,8-tetrahydrochinazolin,
2-(4-Hydroxyphenyl)-6-n-decyl-5,6,7,8-tetrahydrochinazolin,
2-(4-Hydroxyphenyl)-6-(2-methylbutyl)-5,6,7,8-tetrahydrochinazolin,
2-(4-Hydroxyphenyl)-6-(2-ethylhexyl)-5,6,7,8-tetrahydrochinazolin.

(b) Zu einer Lösung von 11,3 g 2-(4-Hydroxyphenyl)-6-n-butyl-5,6,7,8-tetrahydrochinazolin und 4 g Pyridin in 100 ml Toluol werden bei 100° unter Rühren 6,7 g n-Hexanoylchlorid getropft. Nach zweistündigem Nachrühren wird das Reaktionsgemisch noch heiss filtriert, das abfiltrierte Pyridinhydrochlorid mit 50 ml Toluol nachgewaschen und die vereinigten Filtrate eingedampft. Das zurückbleibende 2-(4-n-Hexanoyloxyphenyl)-6-n-butyl-5,6,7,8-tetrahydrochinazolin wird aus Ethylacetat umkristallisiert; Ausbeute 15,3 g farblose Kristalle.

Analog werden hergestellt:
2-(4-Acetoxyphenyl)-6-methyl-5,6,7,-tetrahydrochinazolin,
2-(4-Acetoxyphenyl)-6-ethyl-5,6,7,8-tetrahydrochinazolin,
2-(4-Acetoxyphenyl)-6-n-propyl-5,6,7,8-tetrahydrochinazolin,
2-(4-Acetoxyphenyl)-6-n-butyl-5,6,7,8-tetrahydrochinazolin,
2-(4-Acetoxyphenyl)-6-n-pentyl-5,6,7,8-tetrahydrochinazolin,
2-(4-Acetoxyphenyl)-6-n-hexyl-5,6,7,8-tetrahydrochinazolin,
2-(4-Acetoxyphenyl)-6-n-heptyl-5,6,7,8-tetrahydrochinazolin,
2-(4-Acetoxyphenyl)-6-n-octyl-5,6,7,8-tetrahydrochinazolin,

2-(4-Acetoxyphenyl)-6-n-nonyl-5,6,7,8-tetrahydrochinazolin,
2-(4-Acetoxyphenyl)-6-n-decyl-5,6,7,8-tetrahydrochinazolin,
2-(4-Acetoxyphenyl)-6-(2-methylbutyl)-5,6,7,8-tetrahydrochinazolin,
2-(4-Acetoxyphenyl)-6-(2-ethylhexyl)-5,6,7,8-tetrahydrochinazolin,

2-(4-Propionyloxyphenyl)-6-methyl-5,6,7,8-tetrahydrochinazolin,
2-(4-Propionyloxyphenyl)-6-ethyl-5,6,7,8-tetrahydrochinazolin,
2-(4-Propionyloxyphenyl)-6-n-propyl-5,6,7,8-tetrahydrochinazolin,
2-(4-Propionyloxyphenyl)-6-n-butyl-5,6,7,8-tetrahydrochinazolin,
2-(4-Propionyloxyphenyl)-6-n-pentyl-5,6,7,8-tetrahydrochinazolin,
2-(4-Propionyloxyphenyl)-6-n-hexyl-5,6,7,8-tetrahydrochinazolin,
2-(4-Propionyloxyphenyl)-6-n-heptyl-5,6,7,8-tetrahydrochinazolin,
2-(4-Propionyloxyphenyl)-6-n-octyl-5,6,7,8-tetrahydrochinazolin,
2-(4-Propionyloxyphenyl)-6-n-nonyl-5,6,7,8-tetrahydrochinazolin,
2-(4-Propionyloxyphenyl)-6-n-decyl-5,6,7,8-tetrahydrochinazolin,
2-(4-Propionyloxyphenyl)-6-(2-methylbutyl)-5,6,7,8-tetrahydrochinazolin,
2-(4-Propionyloxyphenyl)-6-(2-ethylhexyl)-5,6,7,8-tetrahydrochinazolin,

2-(4-n-Butyryloxyphenyl)-6-methyl-5,6,7,8-tetrahydrochinazolin,
2-(4-n-Butyryloxyphenyl)-6-ethyl-5,6,7,8-tetrahydrochinazolin,
2-(4-n-Butyryloxyphenyl)-6-n-propyl-5,6,7,8-tetrahydrochinazolin,
2-(4-n-Butyryloxyphenyl)-6-n-butyl-5,6,7,8-tetrahydrochinazolin,
2-(4-n-Butyryloxyphenyl)-6-n-pentyl-5,6,7,8-tetrahydrochinazolin,
2-(4-n-Butyryloxyphenyl)-6-n-hexyl-5,6,7,8-tetrahydrochinazolin,
2-(4-n-Butyryloxyphenyl)-6-n-heptyl-5,6,7,8-tetrahydrochinazolin,
2-(4-n-Butyryloxyphenyl)-6-n-octyl-5,6,7,8-tetrahydrochinazolin,
2-(4-Butyryloxyphenyl)-6-n-nonyl-5,6,7,8-tetrahydrochinazolin,
2-(4-n-Butyryloxyphenyl)-6-n-decyl-5,6,7,8-tetrahydrochinazolin,
2-(4-n-Butyryloxyphenyl)-6-(2-methylbutyl)-5,6,7,8-tetrahydrochinazolin,
2-(4-n-Butyryloxyphenyl)-6-(2-ethylhexyl)-5,6,7,8-tetrahydrochinazolin,

2-(4-n-Pentanoyloxyphenyl)-6-methyl-5,6,7,8-tetrahydrochinazolin,
2-(4-n-Pentanoyloxyphenyl)-6-ethyl-5,6,7,8-tetrahydrochinazolin,
2-(4-n-Pentanoyloxyphenyl)-6-n-propyl-5,6,7,8-tetrahydrochinazolin,
2-(4-n-Pentanoyloxyphenyl)-6-n-butyl-5,6,7,8-tetrahydrochinazolin,
2-(4-n-Pentanoyloxyphenyl)-6-n-pentyl-5,6,7,8-tetrahydrochinazolin,
2-(4-n-Pentanoyloxyphenyl)-6-n-hexyl-5,6,7,8-tetrahydrochinazolin,
2-(4-n-Pentanoyloxyphenyl)-6-n-heptyl-5,6,7,8-tetrahydrochinazolin,
2-(4-n-Pentanoyloxyphenyl)-6-n-octyl-5,6,7,8-tetrahydrochinazolin,
2-(4-n-Pentanoyloxyphenyl)-6-n-nonyl-5,6,7,8-tetrahydrochinazolin,
2-(4-n-Pentanoyloxyphenyl)-6-n-decyl-5,6,7,8-tetrahydrochinazolin,
2-(4-n-Pentanoyloxyphenyl)-6-(2-methylbutyl)-5,6,7,8-tetrahydrochinazolin,
2-(4-n-Pentanoyloxyphenyl)-6-(2-ethylhexyl)-5,6,7,8-tetrahydrochinazolin,

2-(4-n-Hexanoyloxyphenyl)-6-methyl-5,6,7,8-tetrahydrochinazolin,
2-(4-n-Hexanoyloxyphenyl)-6-ethyl-5,6,7,8-tetrahydrochinazolin,
2-(4-n-Hexanoyloxyphenyl)-6-n-propyl-5,6,7,-tetrahydrochinazolin,
2-(4-n-Hexanoyloxyphenyl)-6-n-pentyl-5,6,7,8-tetrahydrochinazolin,
2-(4-n-Hexanoyloxyphenyl)-6-n-hexyl-5,6,7,8-tetrahydrochinazolin,
2-(4-n-Hexanoyloxyphenyl)-6-n-heptyl-5,6,7,8-tetrahydrochinazolin,
2-(4-n-Hexanoyloxyphenyl)-6-n-octyl-5,6,7,8-tetrahydrochinazolin,
2-(4-n-Hexanoyloxyphenyl)-6-n-nonyl-5,6,7,8-tetrahydrochinazolin,
2-(4-n-Hexanoyloxyphenyl)-6-n-decyl-5,6,7,8-tetrahydrochinazolin,
2-(4-n-Hexanoyloxyphenyl)-6-(2-methylbutyl)-5,6,7,8-tetrahydrochinazolin,
2-(4-n-Hexanoyloxyphenyl)-6-(2-ethylhexyl)-5,6,7,8-tetrahydrochinazolin,

2-(4-n-Heptanoyloxyphenyl)-6-methyl-5,6,7,8-tetrahydrochinazolin,
2-(4-n-Heptanoyloxyphenyl)-6-ethyl-5,6,7,8-tetrahydrochinazolin,
2-(4-n-Heptanoyloxyphenyl)-6-n-propyl-5,6,7,8-tetrahydrochinazolin,
2-(4-n-Heptanoyloxyphenyl)-6-n-butyl-5,6,7,8-tetrahydrochinazolin,
2-(4-n-Heptanoyloxyphenyl)-6-n-pentyl-5,6,7,8-tetrahydrochinazolin,
2-(4-n-Heptanoyloxyphenyl)-6-n-hexyl-5,6,7,8-tetrahydrochinazolin,
2-(4-n-Heptanoyloxyphenyl)-6-n-heptyl-5,6,7,8-tetrahydrochinazolin,
2-(4-n-Heptanoyloxyphenyl)-6-n-octyl-5,6,7,8-tetrahydrochinazolin,
2-(4-n-Heptanoyloxyphenyl)-6-n-nonyl-5,6,7,8-tetrahydrochinazolin,
2-(4-n-Heptanoyloxyphenyl)-6-n-decyl-5,6,7,8-tetrahydrochinazolin,
2-(4-n-Heptanoyloxyphenyl)-6-(2-methylbutyl)-5,6,7,8-tetrahydrochinazolin,

2-(4-n-Heptanoyloxyphenyl)- 6-(2-ethylhexyl)-5,6,7,8-tetrahydrochinazolin,

2-(4-n-Octanoyloxyphenyl)-6-methyl-5,6,7,8-tetrahydrochinazolin,
2-(4-n-Octanoyloxyphenyl)-6-ethyl-5,6,7,8-tetrahydrochinazolin,
2-(4-n-Octanoyloxyphenyl)-6-n-propyl-5,6,7,8-tetrahydrochinazolin,
2-(4-n-Octanoyloxyphenyl)-6-n-butyl-5,6,7,8-tetrahydrochinazolin,
2-(4-n-Octanoyloxyphenyl)-6-n-pentyl- 5,6,7,8-tetrahydrochinazolin,
2-(4-n-Octanoyloxyphenyl)-6-n-hexyl-5,6,7,8-tetrahydrochinazolin,
2-(4-n-Octanoyloxyphenyl)-6-n-heptyl-5,6,7,8-tetrahydrochinazolin,
2-(4-n-Octanoyloxyphenyl)-6-n-octyl-5,6,7,8-tetrahydrochinazolin,
2-(4-n-Octanoyloxyphenyl)-6-n-nonyl-5,6,7,8-tetrahydrochinazolin,
2-(4-n-Octanoyloxyphenyl)-6-n-decyl-5,6,7,8-tetrahydrochinazolin,
2-(4-n-Octanoyloxyphenyl)-6-(2-methylbutyl)-5,6,7,8-tetrahydrochinazolin,
2-(4-n-Octanoyloxyphenyl)-6-(2-ethylhexyl)-5,6,7,8-tetrahydrochinazolin,

2-(4-Ethoxycarbonyloxyphenyl)-6-methyl-5,6,7,8-tetrahydrochinazolin,
2-(4-Ethoxycarbonyloxyphenyl)-6-ethyl-5,6,7,8-tetrahydrochinazolin,
2-(4-Ethoxycarbonyloxyphenyl)-6-n-propyl-5,6,7,8-tetrahydrochinazolin,
2-(4-Ethoxycarbonyloxyphenyl)-6-n-butyl-5,6,7,8-tetrahydrochinazolin,
2-(4-Ethoxycarbonyloxyphenyl)-6-n-pentyl-5,6,7,8-tetrahydrochinazolin,
2-(4-Ethoxycarbonyloxyphenyl)-6-n-hexyl-5,6,7,8-tetrahydrochinazolin,
2-(4-Ethoxycarbonyloxyphenyl)-6-n-heptyl-5,6,7,8-tetrahydrochinazolin,
2-(4-Ethoxycarbonyloxyphenyl)-6-n-octyl-5,6,7,8-tetrahydrochinazolin,
2-(4-Ethoxycarbonyloxyphenyl)-6-n-nonyl-5,6,7,8-tetrahydrochinazolin,
2-(4-Ethoxycarbonyloxyphenyl)-6-n-decyl-5,6,7,8-tetrahydrochinazolin,
2-(4-Ethoxycarbonyloxyphenyl)-6-(2-methylbutyl)-5,6,7,8-tetrahydrochinazolin,
2-(4-Ethoxycarbonyloxyphenyl)-6-(2-ethylhexyl)-5,6,7,8-tetrahydrochinazolin,

2-(4-n-Propyloxycarbonyloxyphenyl)-6-methyl-5,6,7,8-tetrahydrochinazolin,
2-(4-n-Propyloxycarbonyloxyphenyl)-6-ethyl-5,6,7,8-tetrahydrochinazolin,
2-(4-n-Propyloxycarbonyloxyphenyl)-6-n-propyl-5,6,7,8-tetrahydrochinazolin,
2-(4-n-Propyloxycarbonyloxyphenyl)-6-n-butyl-5,6,7,8-tetrahydrochinazolin,
2-(4-n-Propyloxycarbonyloxyphenyl)-6-n-pentyl-5,6,7,8-tetrahydrochinazolin,
2-(4-n-Propyloxycarbonyloxyphenyl)-6-n-hexyl-5,6,7,8-tetrahydrochinazolin,
2-(4-n-Propyloxycarbonyloxyphenyl)-6-n-octyl-5,6,7,8-tetrahydrochinazolin,
2-(4-n-Propyloxycarbonyloxyphenyl)-6-n-nonyl-5,6,7,8-tetrahydrochinazolin,
2-(4-n-Propyloxycarbonyloxyphenyl)-6-n-decyl-5,6,7,8-tetrahydrochinazolin,
2-(4-n-Propyloxycarbonyloxyphenyl)-6-(2-methylbutyl)- 5,6,7,8-tetrahydrochinazolin,
2-(4-n-Propyloxycarbonyloxyphenyl)-6-(2-ethylhexyl)-5,6,7,8-tetrahydrochinazolin,

2-(4-n-Butyloxycarbonyloxyphenyl)-6-methyl-5,6,7,8-tetrahydrochinazolin,
2-(4-n-Butyloxycarbonyloxyphenyl)-6-ethyl-5,6,7,8-tetrahydrochinazolin,
2-(4-n-Butyloxycarbonyloxyphenyl)-6-n-propyl-5,6,7,8-tetrahydrochinazolin,
2-(4-n-Butyloxycarbonyloxyphenyl)-6-n-butyl-5,6,7,8-tetrahydrochinazolin,
2-(4-n-Butyloxycarbonyloxyphenyl)-6-n-pentyl-5,6,7,8-tetrahydrochinazolin,
2-(4-n-Butyloxycarbonyloxyphenyl)-6-n-hexyl-5,6,7,8-tetrahydrochinazolin,
2-(4-n-Butyloxycarbonyloxyphenyl)-6-n-heptyl-5,6,7,8-tetrahydrochinazolin,
2-(4-n-Butyloxycarbonyloxyphenyl)-6-n-octyl-5,6,7,8-tetrahydrochinazolin,
2-(4-n-Butyloxycarbonyloxyphenyl)-6-n-nonyl-5,6,7,8-tetrahydrochinazolin,
2-(4-n-Butyloxycarbonyloxyphenyl)-6-n-decyl-5,6,7,8-tetrahydrochinazolin,
2-(4-n-Butyloxycarbonyloxyphenyl)-6-(2-methylbutyl)-5,6,7,8-tetrahydrochinazolin,
2-(4-n-Butyloxycarbonyloxyphenyl)-6-(2-ethylhexyl)-5,6,7,8-tetrahydrochinazolin,

2-(4-n-Pentyloxycarbonyloxyphenyl)-6-methyl-5,6,7,8-tetrahydrochinazolin,
2-(4-n-Pentyloxycarbonyloxyphenyl)-6-ethyl-5,6,7,8-tetrahydrochinazolin,
2-(4-n-Pentyloxycarbonyloxyphenyl)-6-n-propyl-5,6,7,8-tetrahydrochinazolin,
2-(4-n-Pentyloxycarbonyloxyphenyl)-6-n-butyl-5,6,7,8- tetrahydrochinazolin,
2-(4-n-Pentaloxycarbonyloxyphenyl)-6-n-pentyl-5,6,7,8-tetrahydrochinazolin,
2-(4-n-Pentyloxycarbonyloxyphenyl)-6-n-hexyl-5,6,7,8-tetrahydrochinazolin,
2-(4-n-Pentyloxycarbonyloxyphenyl)-6-n-heptyl-5,6,7,8-tetrahydrochinazolin,
2-(4-n-Pentyloxycarbonyloxyphenyl)-6-n-octyl-5,6,7,8-tetrahydrochinazolin,
2-(4-n-Pentyloxycarbonyloxyphenyl)-6-n-nonyl-5,6,7,8-tetrahydrochinazolin,
2-(4-n-Pentyloxycarbonyloxyphenyl)-6-n-decyl-5,6,7,8-tetrahydrochinazolin,
2-(4-n-Pentyloxycarbonyloxyphenyl)-6-(2-methylbutyl)-5,6,7,8-tetrahydrochinazolin,
2-(4-n-Pentyloxycarbonyloxyphenyl)-6-(2-ethylhexyl)-5,6,7,8-tetrahydrochinazolin.

2-(4-n-Hexyloxycarbonyloxyphenyl)-6-methyl-5,6,7,8-tetrahydrochinazolin,
2-(4-n-Hexylocycarbonyloxyphenyl)-6-ethyl-5,6,7,8-tetrahydrochinazolin,

2-(4-n-Hexylocycarbonyloxyphenyl)-6-n-propyl-5,6,7,8-tetrahydrochinazolin,
2-(4-n-Hexyloxycarbonyloxyphenyl)-6-n-butyl-5,6,7,8-tetrahydrochinazolin,
2-(4-n-Hexyloxycarbonyloxyphenyl)-6-n-pentyl-5,6,7,8-tetrahydrochinazolin,
2-(4-n-Hexyloxycarbonyloxyphenyl)-6-n-hexyl-5,6,7,8-tetrahydrochinazolin,
2-(4-n-Hexylocycarbonyloxyphenyl)-6-n-heptyl-5,6,7,8-tetrahydrochinazolin,
2-(4-n-Hexyloxycarbonyloxyphenyl)-6-n-octyl-5,6,7,8-tetrahydrochinazolin,
2-(4-n-Hexyloxycarbonyloxyphenyl)-6-n-nonyl-5,6,7,8-tetrahydrochinazolin,
2-(4-n-Hexyloxycarbonyloxyphenyl)-6-n-decyl-5,6,7,8-tetrahydrochinazolin,
2-(4-n-Hexyloxycarbonyloxyphenyl)-6-(2-methylbutyl)-5,6,7,8-tetrahydrochinazolin,
2-(4-n-Hexyloxycarbonyloxyphenyl)-6-(2-ethylhexyl)-5,6,7,8-tetrahydrochinazolin.

Beispiel 4

(a) 16,7 g 2-(4-Cyanophenyl)-6-n-heptyl-5,6,7,8-tetrahydrochinazolin werden mit einer Lösung von 6 g Kaliumhydroxid in 50 ml Ethanol 4 Stunden zum Sieden erhitzt. Anschliessend wird das Reaktionsgemisch in 200 ml Wasser gegossen, die wässrige Lösung mit 10%iger wässriger Salzsäure angesäuert (pH 2–3) und die saure Lösung dreimal mit je 100 ml Diethylether ausgeschüttelt. Die organischen Phasen werden über Calciumchlorid getrocknet und eingedampft. Es bleiben 14 g 2-(4-Carboxyphenyl)-6-n-heptyl-5,6,7,8-tetrahydrochinazolin als grauweisse, kristalline Masse zurück.

Analog werden hergestellt:
2-(4-Carboxyphenyl)-6-methyl-5,6,7,8-tetrahydrochinazolin,
2-(4-Carboxyphenyl)-6-ethyl-5,6,7,8-tetrahydrochinazolin,
2-(4-Carboxyphenyl)-6-n-propyl-5,6,7,8-tetrahydrochinazolin,
2-(4-Carboxyphenyl)-6-n-butyl-5,6,7,8-tetrahydrochinazolin,
2-(4-Carboxyphenyl)-6-n-pentyl-5,6,7,8-tetrahydrochinazolin,
2-(4-Carboxyphenyl)-6-n-hexyl-5,6,7,8-tetrahydrochinazolin,
2-(4-Carboxyphenyl)-6-n-octyl-5,6,7,8-tetrahydrochinazolin,
2-(4-Carboxyphenyl)-6-n-nonyl-5,6,7,8-tetrahydrochinazolin,
2-(4-Carboxyphenyl)-6-n-decyl-5,6,7,8-tetrahydrochinazolin,
2-(4-Carboxyphenyl)-6-(2-methylbutyl)-5,6,7,8-tetrahydrochinazolin,
2-(4-Carboxyphenyl)-6-(2-ethylhexyl)-5,6,7,8-tetrahydrochinazolin.

(b) 7 g 2-(4-Carboxyphenyl)-6-n-heptyl-5,6,7,8-tetrahydrochinazolin werden mit 15 g Schwefeltetrafluorid im Bombenrohr 8 Stunden auf 100° erwärmt. Nach dem Abkühlen wird das überschüssige Schwefeltetrafluorid durch Einblasen von Stickstoff entfernt und das zurückbleibende 2-(4-Trifluormethylphenyl)-6-n-heptyl-5,6,7,8-tetrahydrochinazolin aus Ethanol umkristallisiert; Ausbeute 4,8 g farblose Kristalle.

Analog werden hergestellt:
2-(4-Trifluormethylphenyl)-6-methyl-5,6,7,8-tetrahydrochinazolin,
2-(4-Trifluormethylphenyl)-6-ethyl-5,6,7,8-tetrahydrochinazolin,
2-(4-Trifluormethylphenyl)-6-n-propyl-5,6,7,8-tetrahydrochinazolin,
2-(4-Trifluormethylphenyl)-6-n-butyl-5,6,7,8-tetrahydrochinazolin,
2-(4-Trifluormethylphenyl)-6-n-pentyl-5,6,7,8-tetrahydrochinazolin,
2-(4-Trifluormethylphenyl)-6-n-hexyl-5,6,7,8-tetrahydrochinazolin,
2-(4-Trifluormethylphenyl)-6-n-octyl-5,6,7,8-tetrahydrochinazolin,
2-(4-Trifluormethylphenyl)-6-n-nonyl-5,6,7,8-tetrahydrochinazolin,
2-(4-Trifluormethylphenyl)-6-n-decyl-5,6,7,8-tetrahydrochinazolin,
2-(4-Trifluormethylphenyl)-6-(2-methylbutyl)-5,6,7,8-tetrahydrochinazolin,
2-(4-Trifluormethylphenyl)-6-(2-ethylhexyl)-5,6,7,8-tetrahydrochinazolin.

Die folgenden Beispiele betreffen flüssigkristalline Dielektrika nach der Erfindung:

Beispiel 5

Eine Mischung aus 40 Gewichtsteilen 4-(trans-4-n-Propylcyclohexyl)-benzonitril und 60 Gewichtsteilen 4-(trans-4-n-Pentylcyclohexyl)-benzonitril hat eine nematische Phase im Temperatubereich von +8° bis +51°. Durch Zusatz von 22 Gewichtsteilen 2-(4-n-Butyloxyphenyl)-6-n-hexyl-5,6,7,8-tetrahydrochinazolin wird ein ternäres flüssigkristallines Dielektrikum mit einer nematischen Phase im Temperaturbereich von +2° bis +60° erhalten.

Beispiel 6

Eine Mischung aus 35 Gewichtsteilen 4-(trans-4-n-Propylcyclohexyl)-benzonitril und 65 Gewichtsteilen 4-n-Pentyl-4'-cyanobiphenyl hat eine nematische Phase im Temperaturbereich von +3° bis +39°. Durch Zusatz von 19 Gewichtsteilen 2-(4-n-Butyloxyphenyl)-6-n-hexyl-5,6,7,8-tetrahydrochinazolin wird ein flüssigkristallines Dielektrikum erhalten, das ein ternäres Eutektikum mit einer nematischen Phase im Temperaturbereich von − 3° bis +48° darstellt.

Beispiel 7

Eine Mischung aus 30 Gewichtsteilen 4-(trans-4-n-Butylcyclohexyl)-benzonitril, 55 Gewichtsteilen Anissäure-4-n-pentylphenylester und 15 Gewichtsteilen 4-(trans-4-n-Pentylcyclohexyl)-4'-cyanobiphenyl hat eine nematische Phase im Temperatubereich von +6° bis +64° und eine DKA von +8,1. Durch Zusatz von 12 Gewichtsteilen 2-(4-Cyanophenyl)-6-n-hexyl- 5,6,7,8-tetrahydrochinazolin wird ein flüssigkristallines Dielektrikum erhalten, das ein quaternäres Eutektikum mit einer

nematischen Phase im Temperatubereich von +2° bis +67° und einer DKA von +9,3 ist.

Beispiel 8

Eine Mischung aus 31 Gewichtsteilen 4-(trans-4-n-Propylcyclohexyl)-1-n-butyloxybenzol, 33 Gewichtsteilen 4-n-Hexyloxybenzoesäure-4'-n-pentylphenylester und 36 Gewichtsteilen 4-n-Hexanoyloxybenzoesäure-4'-n-heptylphenylester hat eine nematische Phase im Temperaturbereich von +6° bis +48°, eine DKA von −0,2 und eine optische Anisotropie von +0,12. Durch Zusatz von 10 Gewichtsteilen 2-(4-Cyanophenyl)-6-n-pentyl-5,6,7,8-tetrahydrochinazolin wird ein flüssigkristallines Dielektrikum mit einer nematischen Phase im Temperaturbereich von +3° bis +51°, einer DKA von +2,5 und einer optischen Anisotropie von +0,13 erhalten.

**Patentansprüche**

1. Tetrahydrochinazoline der Formel (I)

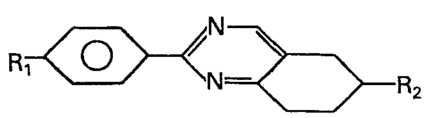
(I)

worin $R_1$ Alkyl, Alkoxy, Alkanoyloxy oder Alkoxycarbonyloxy mit jeweils bis zu 8 Kohlenstoffatomen, CN, $CF_3$, Halogen oder $NO_2$ und $R_2$ Alkyl mit bis zu 10 Kohlenstoffatomen bedeutet.

2. Tetrahydrochinazoline nach Anspruch 1 der Formel (I), worin $R_1$ CN ist.

3. Tetrahydrochinazoline nach Anspruch 1 der Formel (I), worin $R_1$ Alkyl, Alkoxy oder Alkanoyloxy mit bis zu 6 Kohlenstoffatomen ist.

4. Verfahren zur Herstellung von Tetrahydrochinazolinen der Formel (I)

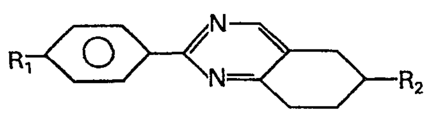
(I)

worin $R_1$, Alkyl, Alkoxy, Alkanoyloxy oder Alkoxycarbonyloxy mit jeweils bis zu 8 Kohlenstoffatomen, CN, $CF_3$, Halogen oder $NO_2$ und $R_2$ Alkyl mit bis zu 10 Kohlenstoffatomen bedeutet, dadurch gekennzeichnet, dass man eine Verbindung der Formel (II)

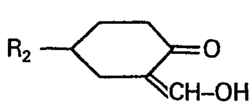
(II)

worin $R_2$ die vorstehend angegebene Bedeutung besitzt, in Gegenwart eines basischen Kondensationsmittels mit einer Verbindung der Formel (III) umsetzt

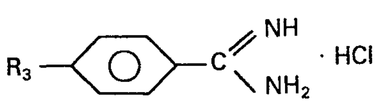
(III)

worin $R_3$ Alkyl oder Alkoxy mit bis zu 8 C-Atomen, Benzyloxy, Halogen oder $NO_2$ bedeutet, und gewünschtenfalls in der dabei erhaltenen Verbindung der Formel (IV)

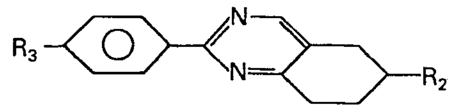
(IV)

den Rest $R_3$ in an sich bekannter Weise in einen davon verschiedenen Rest $R_1$ umwandelt.

5. Verwendung eines Tetrahydrochinazolins der Formel (I)

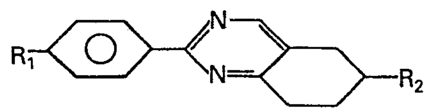
(I)

worin $R_1$, Alkyl, Alkoxy, Alkanoyloxy oder Alkoxycarbonyloxy mit jeweils bis zu 8 Kohlenstoffatomen, CN, $CF_3$, Halogen oder $NO_2$ und $R_2$ Alkyl mit bis zu 10 Kohlenstoffatomen bedeutet, als Komponente eines flüssigkristallinen Dielektrikums.

6. Flüssigkristallines Dielektrikum, dadurch gekennzeichnet, dass es 1 bis 50 Gewichtsprozent eines Tetrahydrochinazolins der Formel (I) enthält,

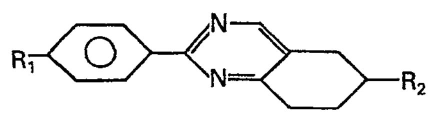
(I)

worin $R_1$ Alkyl, Alkoxy, Alkanoyloxy oder Alkoxycarbonyloxy mit jeweils bis zu 8 Kohlenstoffatomen, CN, $CF_3$, Halogen oder $NO_2$ und $R_2$ Alkyl mit bis zu 10 Kohlenstoffatomen bedeutet.

7. Elektrooptisches Anzeigeelement auf der Basis einer Flüssigkristallzelle, dadurch gekennzeichnet, dass es ein flüssigkristallines Dielektrikum nach Anspruch 6 enthält.

**Revendications**

1. Tétrahydroquinazolines de formule (I)

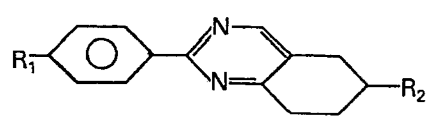
(I)

dans laquelle $R_1$ représente un radical alkyle, alcoxy, alcanoyloxy ou alcoxycarbonyloxy avec jusqu'à 8 atomes de carbone, CN, $CF_3$, halogène ou $NO_2$ et $R_2$ est un radical alkyle avec jusqu'à 10 atomes de carbone.

2. Tétrahydroquinazolines selon la revendication 1 de formule (I), où $R_1$ est CN.

3. Tétrahydroquinazolines selon la revendication 1 de formule (I), dans laquelle $R_1$ est un radical alkyle, alcoxy ou alcanoyloxy avec jusqu'à 6 atomes de carbone.

4. Procédé pour la fabrication des tétrahydroquinazolines de formule (I)

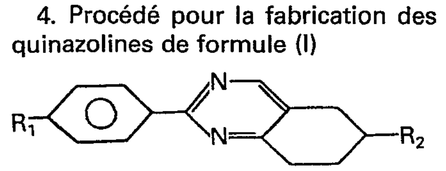
(I)

dans laquelle $R_1$ représente un radical alkyle, alcoxy, alcanoyloxy ou alcoxycarbonyloxy avec respectivement jusqu'à 8 atomes de carbone, CN, $CF_3$, halogène ou $NO_2$ et $R_2$ est un radical alkyle avec jusqu'à 10 atomes de carbone, caractérisé par le fait qu'on fait réagir une combinaison de formule (II)

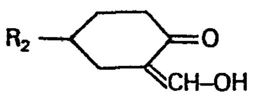

(II)

dans laquelle $R_2$ a la signification indiquée ci-dessus, en présence d'un agent de condensation basique, avec une combinaison de la formule (III)

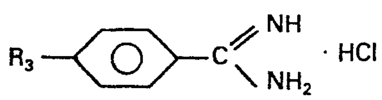

(III)

dans laquelle $R_3$ représente un radical alkyle ou alcoxy avec jusqu'à 8 atomes de carbone, benzyloxy, halogène ou $NO_2$ et, le cas échéant, on transforme, dans la combinaison obtenue alors de formule (IV)

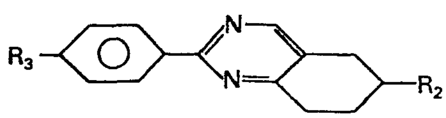

(IV)

le radical $R_3$ d'une manière connue en un radical $R_1$ différent de celui-ci.

5. Utilisation d'une tétrahydroquinazoline de formule (I)

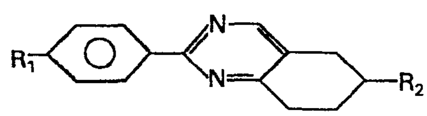

(I)

dans laquelle $R_1$ représente un radical alkyle, alcoxy, alcanoyloxy ou alcoxycarbonyloxy avec respectivement jusqu'à 8 atomes de carbone, CN, $CF_3$, halogène ou $NO_2$ et $R_2$ est un radical alkyle avec jusqu'à 10 atomes de carbone, comme composant d'un diélectrique à cristaux liquides.

6. Diélectrique à cristaux liquides caractérisé par le fait qu'il contient jusqu'à 1 à 50% en poids d'une tétrahydroquinazoline de formule (I),

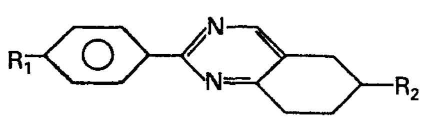

(I)

dans laquelle $R_1$ représente un radical alkyle, alcoxy, alcanoyloxy ou alcoxycarbonyloxy avec respectivement jusqu'à 8 atomes de carbone, CN, $CF_3$, halogène ou $NO_2$ et $R_2$ est un radical alkyle avec jusqu'à 10 atomes de carbone.

7. Elément d'affichage électro-optique à base d'une cellule de cristaux liquides, caractérisé par le fait qu'il renferme un diélectrique à cristaux liquides selon la revendication 6.

**Claims**

1. Tetrahydroquinazolines of the formula (I)

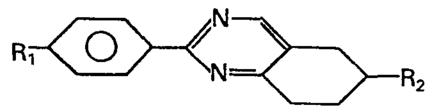

(I)

wherein $R_1$ denotes alkyl, alkoxy, alkanoyloxy or alkoxycarbonyloxy with up to 8 carbon atoms in each case, CN, $CF_3$, halogen or $NO_2$ and $R_2$ denotes alkyl with up to 10 carbon atoms.

2. Tetrahydroquinazolines according to Claim 1 of the formula (I), wherein $R_1$ is CN.

3. Tetrahydroquinazolines according to Claim 1 of the formula (I), wherein $R_1$ is alkyl, alkoxy or alkanoyloxy having up to 6 carbon atoms.

4. Process for the manufacture of tetrahydroquinazolines of the formula (I)

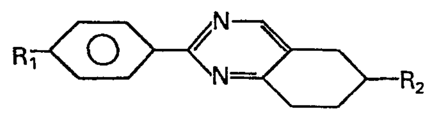

(I)

wherein $R_1$ denotes alkyl, alkoxy, alkanoyloxy or alkoxycarbonyloxy with up to 8 carbon atoms in each case, CN, $CF_3$, halogen or $NO_2$ and $R_2$ denotes alkyl with up to 10 carbon atoms, characterised in that a compound of the formula (II)

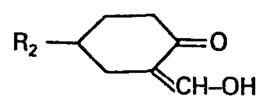

(II)

wherein $R_2$ has the meaning indicated above, is reacted, in the presence of a basic condensation agent, with a compound of the formula (III)

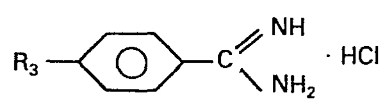

(III)

wherein $R_3$ denotes alkyl or alkoxy having up to 8 C atoms, benzyloxy, halogen or $NO_2$, and, if desired, the radical $R_3$ in the compound, thus obtained, of the formula (IV)

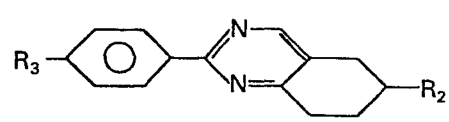

(IV)

is converted in a manner known per se to a radical $R_1$ which differs from $R_3$.

5. Use of a tetrahydroquinazoline of the formula (I)

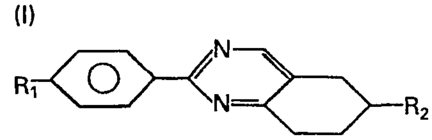

(I)

wherein $R_1$ denotes alkyl, alkoxy, alkanoyloxy or alkoxycarbonyloxy with up to 8 carbon atoms in each case, CN, $CF_3$, halogen or $NO_2$ and $R_2$ de-

notes alkyl with up to 10 carbon atoms, as a component of a liquid-crystalline dielectric.

6. LIquid-crystalline dielectric, characterised in that it contains 1 to 50 per cent by weight of a tetrahydroquinazoline of the formula (I)

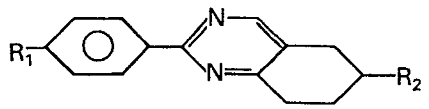

(I)

wherein $R_1$ denotes alkyl, alkoxy, alkanoyloxy or alkoxycarbonyloxy with up to 8 carbon atoms in each case, CN, $CF_3$, halogen or $NO_2$ and $R_2$ denotes alkyl with up to 10 carbon atoms.

7. Electro-optical display element based on a liquid crystal cell, characterised in that it contains a liquid-crystalline dielectric according to Claim 6.